(19) Europäisches Patentamt
European Patent Office
Office européen des brevets

(11) **EP 1 394 716 A1**

(12) **EUROPEAN PATENT APPLICATION**

(43) Date of publication:
**03.03.2004 Bulletin 2004/10**

(51) Int Cl.$^7$: **G06F 19/00**

(21) Application number: **02447151.8**

(22) Date of filing: **07.08.2002**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(71) Applicant: **Université Libre de Bruxelles-Hôpital Erasme**
**1070 Bruxelles (BE)**

(72) Inventors:
• **Venet, David**
**1180 Bruxelles (BE)**

• **Maenhaut, Carine Estelle**
**1420 Braine L'Alleud (BE)**
• **Bersini, Hugues**
**1170 Bruxelles (BE)**

(74) Representative:
**Brants, Johan Philippe Emile et al**
**De Clercq, Brants & Partners cv**
**Edgard Gevaertdreef 10a**
**9830 Sint-Martens-Latem (BE)**

Remarks:
Claims 11 - 24 are deemed to be abandoned due to non-payment of the claims fees (Rule 31 (2) EPC).

(54) **Method to avoid signals-saturation in the quantification of polynucleotide microarrays, a device, a computer program and data therefor**

(57) The invention relates to a method for avoiding signal-saturation in the quantification of polynucleotide microarrays, a device, a computer program and data therefor. The invention quantitatively measures an optical property of a set of samples, said optical property relating to the concentration of the macromolecule to be determined, using an optical detector at two or more different detector gains. The invention uses an algorithm comprising 1) an equation which relates the real intensity to detector gain and measured intensity and 2) minimisation of said equation, and 3) two data matrices which are iteratively modified so as to exclude samples which are saturated, as judged by their predicted intensity. Each iteration of the algorithm improves the prediction and the matrices are concomitantly modified so as to re-include or exclude samples which are saturated. The invention discloses initialisation and normalisation procedures.

FIGURE 1

EP 1 394 716 A1

## Description

### Field of the Invention

The present invention relates to a method for measuring samples using an optical detector so as to avoid signal saturation. It further relates to a device implementing the method. It further relates to a computer program as used in the method. It further relates to data produced by the method, computer program or device.

### Background of the Invention

Microarray technology allows for the quantification of thousands of samples simultaneously. In its most common form, samples are individually spotted onto a slide and a detecting device - usually an optical reader - scans the slide by mechanically moving the slide or detector to enable one sample or a row of samples at a time to be measured. There are situations where the range of concentration of samples on the same slide can vary widely. For example, where levels of gene expression are measured by detecting mRNA concentrations (usually *via* reversed-transcribed cDNA), the detector may be required to measure concentrations ranging from 1 molecule per sample to more than 10,000 molecules per sample. The wide array of concentrations presents dynamic range problems for the detecting device - molecules present in a low concentration would give a weak-signal accompanied by noise, while those present at high concentrations would saturate the detector. Manual intervention for observing and correcting the problem is not feasible since a single slide can contain up to 65,000 samples.

One type of optical detector which currently forms the state of the art comprises a photomultiplier tube which produces an analogue output, the magnitude of which corresponds to the intensity of a sample. Thus the measured intensity of a sample is obtained directly from the output of the photomultiplier tube. Another type of optical detector is a scanning device comprising a photomultiplier tube which scans a sample so as to produce a digitised image of a sample. Thus a single sample is represented by an image comprising a grid of picture elements (pixels), the intensity of each pixel of said image being equivalent to a value of the analogue output from the photomultiplier tube. To obtain a measured intensity value for a single sample recorded as a digitised image, the image is analysed by software which determines the boundaries of the sample and calculates the mean intensity value of the sample within the boundaries. Using either type of optical detector, a measured intensity value for each sample is obtained.

The current method for measuring saturated samples is to vary the gain of the detector *i.e.* change its sensitivity so that the most concentrated sample appears just below the saturation limit of the detector. The method requires reading the slide at several different detector gains in the higher range, and analysing the intensity of each sample or pixel until a suitable gain is found which does not lead to detector saturation for any of the samples. The method cannot easily be automated and the chosen gain favours quantification of the more concentrated samples while the lower concentration samples are too weak to be accurately measured.

Another current method for dealing with a set of sample data containing saturated intensity measurements is to ignore measured intensity values above a certain threshold based on the known limits of the detector (e.g. above 40,000). The threshold needs to be sufficiently low to ensure all the retained data is valid and consequently many well-measured samples are discarded.

A method for dealing with saturated/weak-signals due to fluorescently labelled samples detected during electrophoresis, in particular of a sequencing reaction, is disclosed in US Patent No. 5,786,142. The method describes the use of signal integration to increase the dynamic range of the detector; short integration times for detecting high levels of fluorescence and long integration times for detecting low levels of fluorescence. The patent does not describe how to quantify and correlate data collected with short- and long-integration times or how to define the length of the integration times.

US Patent No. 6,171,793 describes a method for scanning a gene probe array using fluorescence in cases where the data dynamic range is greater than that of the detector. In the preferred embodiment, each sample in the array is irradiated with light of a wavelength corresponding to the fluorophore and emissions are detected at two wavelengths. The first wavelength is chosen to fully represent data in the high-intensity proportion of the array, and the second is chosen to fully represent data in the low-intensity proportion of the array. Readings at either wavelength are proportional to each other within the range of concentrations that give valid data. Valid portions of both data sets are combined by scaling one set to the other, and using an appropriate curve fitting algorithm, a scale factor correlation function is calculated which is used to extrapolate the data.

The method disclosed in US Patent No. 6,171,793 does not automate the process of scanning since, for the method to work, *a priori,* saturated or weak-signal measured intensity values have to be excluded from all calculations used to derive the scale factor correlation function. The patent does not disclose a method for automatically excluding saturated or weak-signal data sets, therefore, said data would need to be screened by the operator, using his own means. Furthermore, the use of two different wavelengths to modulate the detection of the fluorescence emission would be problematic when the sample contains more than one fluorophore each having a different and overlapping emission/absorption profile. 'Crosstalk' between the fluorophores would be detrimental to accuracy of each reading.

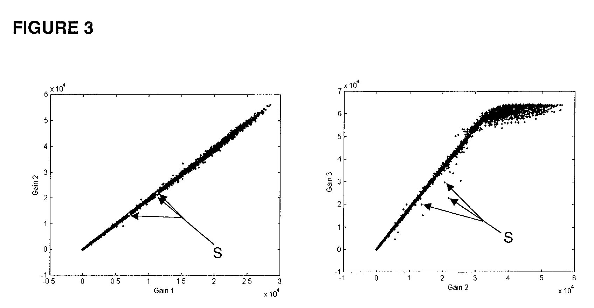

In creating a set of valid measured intensity values, it is often not sufficient to use only measured intensity values that appear below the known upper limit of the detector. This is because a sample may be saturated even when its measured intensity is relatively low. This type of saturation arises in scanning optical detectors which record a digitised image of a sample, when only part of the sample (some pixels) is saturated. The measured intensity of such a sample, being the mean of the saturated and non-saturated parts, exhibits saturated-type behaviour as a function of detector gain. However, the intensity at which the sample saturates is below the upper limit of the detector. Figure 3 shows the results of scanning a microarray of samples using three different gains - G1, G2 and G3 where G1 < G2 < G3. The three samples labelled "S" are saturated as indicated by the fact that they are more on the right of the diagonal line in G2 *vs* G3. The fact they appear on the diagonal line in G1 *vs* G2 indicates that they are not artefacts. Using the detector threshold to determine valid measured intensity values would lead to the inclusion of such invalid data.

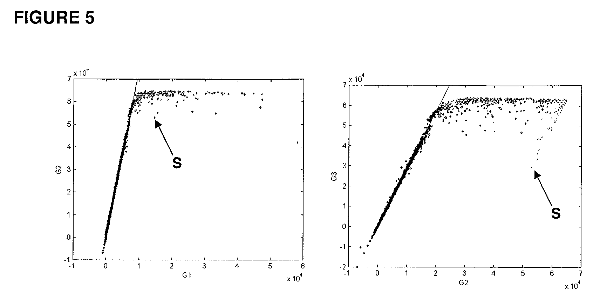

A further problem in creating a data set of valid measurements is that, at high-gain detection, some samples can contaminate their backgrounds. This leads to a sample having a measured intensity which is lower than that measured at a lower gain. Figure 5 shows the results of scanning a microarray of samples using three different gains - G1, G2 and G3 where G1 < G2 < G3. Some samples are detector saturated at G2 and G3 as indicated by the plateau of spots. Several samples, however, including the one indicated by the spot marked 'S' have measured intensity value which is lower at G3 than in G2. Spot 'S' has a measured intensity at G1 of 15,000, and a measured intensity at G2 of 53,000 and surprisingly a measured intensity of 29,000 at G3. Thus, as saturated samples can have a measured intensity below the upper known limit of the detector, screening for valid data points by using the known upper limit of the detector can lead to the inclusion of saturated samples.

## Definitions

*Sample* - a mixture or solution comprising one or more substance of interest, said substance having an optical property that may be quantitatively measured. Examples of substances of interest include DNA, RNA, protein and fluorescently-labelled macromolecules.

*Optical reader/detector* - a device which at some stage quantatively detects visible light *e.g.* scanning fluorimeter, . The terms optical reader and optical detector are interchangeable.

*Detector gain, aj* - the gain used by the optical detector during a scan. In equation [1] below, the value $a_j$ is the detector gain measured during scan $j$ (where is a value $j$ = 1 to $n$; $j$ = 1 has the lowest gain, and $j$ = $n$ has the highest gain). Note - $a_i$ does not have the same value as that entered into the optical reader to set the gain. It is an experimentally-derived unknown.

*Measured Intensity, Mij* - intensity of a sample as measured by an optical detector. It can be proportional to the concentration of the sample or it can be saturated. In equation [1] used below, the value $M_{ij}$ is the measured intensity of spot $i$, measured during scan $j$ (where is a value $j$ = 1 to $n$; $j$ = 1 has the lowest detector gain, and $j$ = $n$ has the highest detector gain)

*Real intensity, vi* - intensity of a sample as measured by an optical detector which is independent of the detector gain. In equation [1] below, $v_i$ is the value of the real intensity of sample i. Thus, for example, when the $a_j$ =1, $M_{ij}$ = 2, $v_i$ = 2; when $a_j$ = 2, $M_{ij}$ = 4, $v_i$ = 2. The real intensity value of a sample is the desired outcome of the invention.

*Predicted intensity* - intensity of a sample expected for a given detector gain under ideal conditions (e.g. non-detector saturating conditions).

*Saturated sample* - a sample whose measured intensity exceeds the limit of the detector at a given gain or a sample whose measured intensity is below that of the limit of the detector, yet the sample is saturated as described above. Related words: saturated sample data.

*Outliers* - measured intensity values which deviate from the predicted value either due to saturation or error.

$$M_{ij} = v_i a_j + b_j \qquad [1]$$

## Objectives of the invention

There is a need for a method that simplifies and automates the process of collecting and analysing data from an optical reader wherein said data has a large dynamic range. Methods in the prior art that recognise the problem require manual interventions by the skilled operator and the expenditure of valuable operator time. Prior art methods provide no means to screen accurately for saturated data, other than by excluding measurements taken from samples where the intensity is at the known limit for the detector. As described above, such screening can lead to the inclusion of saturated measured intensity values which have a low intensity, and hence a distortion of results. The present invention is a method for automatically collecting and analysing a set of optically measured samples whose data dynamic range is large, by measuring the data at more than two detector gains wherein said gains are preferably close to the higher and lower limits for samples, and analysing said data using an algorithm to identify all types of saturated samples and the said algorithm to extract valid data using iterative least-squares fitting minimisation procedures. It can be applied to any programmable optical reader in combination with a computing device with a computer readable medium.

**Summary of the Invention**

A method to quantitate a set of samples using an optical reader, the dynamic range of said set of samples being greater than that of the said optical reader, by measuring intensities of said set at two or more detector gains wherein said gains are preferably close to the higher and lower limits for the said set, and by analysing said measured intensity values using an algorithm to discard all types of saturated sample data, to retain sample data close to saturation and to calculate real intensity values for each sample. Thus, the invention automates the process of removing all types of saturated sample data, it makes a distinction between saturated- and close-to-saturated data, and it calculates real intensity values.

By ' higher and lower limits for samples' it is meant that the lowest gain should be chosen such that no measured intensity value is saturated and the highest gain chosen such that all samples have a measured intensity value above the noise. In practice, the levels may be determined by performing initial measures at very high and very low gains on a set of samples whose real-intensity values have a wide dynamic range in order to establish the said lowest and highest gains. Once the levels have been established for a particular optical detector, they may be applied to all samples thereafter. Alternatively, the said highest and lowest gains may be chosen without prior experimentation, but based on the knowledge the skilled operator has of the limits of the detector.

**Detailed Description of the Invention**

One embodiment of the invention relates to the method above wherein the said algorithm comprises iterative least-squares minimisations to predict said measured intensity values.

Another embodiment of the invention relates to the methods above wherein said algorithm comprises a comparison procedure to discard all types of saturated data and/or non-predicted data from calculations to determine the real intensity values for each sample.

Another embodiment of the invention relates to the methods above wherein the algorithm comprises an iterative routine comprising an equation, said equation relating measured intensity to real intensity and to detector gain using constants, said algorithm comprising the following steps:

a) calculating predicted intensities of samples using said equation and last calculated real intensity values, detector gains and constants, and;

b) using a least-squares minimisation of said equation to determine the detector gain and constants, said minimisation using as input variables the measured intensity values and last-calculated real intensity values, said minimisation also excluding samples judged to have measured intensity values different from the predicted intensity values of step b), said judgment based on stringent criteria.

c) using a least-squares minimisation of said equation to determine the real intensity values, said minimisation using as input variables the measured intensity values and last-calculated detector gain and constants of step b), said minimisation also excluding samples judged to have measured intensity values different from the predicted intensity values of step b), said judgment based on less-stringent criteria.

d) steps a) to d) repeated until change in the constants and real intensity value between iterations is within a predetermined limit.

Another embodiment of the invention relates to the methods above wherein the said algorithm comprises equation [1] -

$$M_{ij} = v_i a_j + b_j \qquad [1]$$

-    in which $M_{ij}$ is the measured intensity of the sample $i$, measured in scan $j$, $v_i$ is the real intensity of sample $i$, $a_j$ is

the detector gain used during scan *j* and *bj* is the scanner offset of scan *j*.

Another embodiment of the invention relates to the methods above wherein the said algorithm comprises a first matrix to determine values of *aj* and *bj,* and a second matrix to determine values of *vi,* said matrices identical in dimensions to *Mij* and the said algorithm comprising the following steps:

a) use of equation [1] and the last calculated values of *aj*, *bj* and *vi* to predict measured intensity values, and;
b) where *Mij* is similar to predicted intensity value of step a), set the corresponding element of the first matrix to one, otherwise to zero, said similarity judged using stringent criteria, and;
c) where *Mij* is similar to predicted intensity value of step a), set the corresponding element of the second matrix to one, otherwise to zero, said similarity judged using less-stringent criteria, and;
d) least-squares minimisation of said equation to determine *aj* and *bj,* said minimisation using as input variables *Mij* and the last-calculated *vi,* said minimisation excluding or including the measured intensity values determined according to the criteria of b) by comprising a multiplication step involving the corresponding element of the first matrix, and;
e) least-squares minimisation of said equation to determine *vi,* said minimisation using as input variables *Mij* and the last-calculated values of *aj and bj,* said minimisation excluding or including the measured intensity values determined according to the criteria of c) by comprising a multiplication step involving the corresponding element of the second matrix, and;
f) repetition of steps a) to f) until change in *aj*, *bj* and *vi* between iterations falls within predefined limits.

Another embodiment of the invention relates to the methods above wherein initial values of *aj*, *bj* and *vi* are calculated using an initialisation routine comprising the following steps:

a) measured intensity values, *Mij,* are optionally filtered to remove those at the known limit for the detector and are used in the proceeding steps, and
b) initial values of the real intensity *vi,* are set to the of values *Mij* measured with the lowest detector gain *i.e. vi = $M_{i1}$*, and
c) initial values of *vi* from step b), and the values of *Mij* are used to calculate the values of *aj* and *bj* by the least-squares minimisation of the equation [1], and
d) the values of *aj* and *bj* calculated in step c) and the values of *Mij* are used to calculate the values of *vi* by the least-squares minimisation of the equation [1], and
e) the so-calculated initial values of *aj*, *bj* and *vi* are used in the first iteration of the iterative routine.

Another embodiment of the invention relates to the methods above wherein the values of *aj* and *bj* determined during the said iterative routine are normalised after the first iteration and each subsequent iteration by dividing *aj* by $a_1$ and by substracting *aj*.$b_1$ from *bj,* respectively.

Another embodiment of the invention relates to the methods above wherein the values of *aj*, *bj* and *vi* determined during the said iterative-routine are normalised after the first and each subsequent iteration by dividing *aj* by $a_1$, and by adding *bj* to min(*v*).*aj*, and by substracting min(*v*) from *vi,* respectively.

Another embodiment of the invention relates to the methods above wherein step of judging similarity between said predicted intensity values and measured intensity values comprises the following steps:

a) values of *vi* are calculated using only columns of *Mik* for values of k between *k* = 1 and *k* = *j* - 1, where *j* (*j*>1) is a column of data, said calculation performed using the iterative-least-squares fitting, result of said calculation is *vi(j)*, and

b) Predicted intensity values are calculated using equation [2] -

$$P_{ij} = a_j v_i(j) + b_j \qquad [2]$$

- in which *aj*, *vi(j)* and *bj* are defined above, and *Pij* is the predicted intensity value of sample *i,* recorded in scan *j*, and

c) Using MATRIX *NSij,* the median error is calculated using equation [3] -

$$ME = \underset{i\,|\,NS(i,j)=1}{\mathrm{median}}\left(\left|P_{ij} - M_{ij}\right|\right) \qquad [3]$$

- in which $Mij$ and $NSij$ and $Pij$ are defined above and $ME$ is the median error, and

d) If $ME$ is greater than 500, $ME$ is set to 500, and

e) Using equation [4] -

$$NS_{ij} = P_{ij} < (M_{ij} + d.ME(1 + e.NS_{ij})) \qquad [4]$$

- in which $NSij$, $Pij$ and $ME$ are defined above, $d$ is a value in the range 1 to 30, $e$ is a value in the range 0 to 1, element $NSij$ of MATRIX $NSij$ is set to zero if the predicted value is much higher than the real value compared to $ME$, or is set to one otherwise, and

f) Using equation [5] -

$$NS2_{ij} = |P_{ij} - M_{ij}| < f.ME(1 + g.NS2_{ij}) \qquad [5]$$

- in which $NS2ij$, $Pij$ and $ME$ are defined above, $f$ is a value in the range 1 to 30, $g$ is a value in the range 0 to 1, element $NS2ij$ of MATRIX $NS2ij$ is set to zero if the predicted value largely differs from the real value compared to $ME$, or is set to one otherwise.

Another embodiment of the invention relates to the methods above wherein said optical reader is a fluorescence micro-array scanner.

Another embodiment of the invention relates to the methods above wherein the said samples are fluorescently labelled DNA probes hybridized onto cDNA attached to a solid support.

Another embodiment of the invention relates to the use of a computer program comprising a computing routine (s), stored on a computer readable medium for transforming a set of wide dynamic-range sample data measured by optical means at two or more different detector gains, said gains preferably close to the higher and lower limits for the set of samples, the said data held as a file in any format and/or as data passed from another computing routine(s), using the said algorithm, to a new data set that corresponds to real intensity values, the results given as readable output and/or a file of any format and/or data passed to other computing routine(s).

Another embodiment of the invention relates to a computer program comprising a computing routine(s), stored on a computer readable medium for transforming a set of wide dynamic-range sample data measured by optical means at two or more different detector gains, said gains preferably close to the higher and lower limits for the set of samples, the said data held as a file in any format and/or as data passed from another computing routine(s), using an algorithm, the said algorithm removing all types of saturated sample data, retaining close-to-saturated sample data and calculating real intensity values for each sample, the said transformation resulting in a new data set that corresponds to real intensity values, the results given as readable output and/or a file of any format and/or data passed to other computing routine(s).

Another embodiment of the invention relates to the computer program above wherein said algorithm of the said computing routine(s), stored on a computer readable medium, comprises an iterative routine comprising an equation, said equation relating measured intensity to real intensity and to detector gain using constants, said algorithm comprising the following steps:

a) calculating predicted intensities of samples using said equation and last calculated real intensity values, detector gains and constants, and;

b) using a least-squares minimisation of said equation to determine the detector gain and constants, said minimisation using as input variables the measured intensity values and last-calculated real intensity values, said minimisation also excluding samples judged to have measured intensity values different from the predicted intensity values of step b), said judgment based on stringent criteria.

c) using a least-squares minimisation of said equation to determine the real intensity values, said minimisation using as input variables the measured intensity values and last-calculated detector gain and constants of step b), said minimisation also excluding samples judged to have measured intensity values different from the predicted intensity values of step b), said judgment based on less-stringent criteria.

d) steps a) to d) repeated until change in the constants and real intensity value between iterations is within a predetermined limit.

Another embodiment of the invention relates to the computer program above wherein the said equation said algorithm of the said computing routine(s), stored on a computer readable medium takes the form of equation [1] -

$$M_{ij} = v_i a_j + b_j \qquad [1]$$

in which $M_{ij}$ is the measured intensity of the sample $i$, measured in scan $j$ (for scans $j = 1$ to n, where the scan $j = 1$ has the lowest detector gain and $j = n$ has the highest detector gain), $v_i$ is the real intensity of spot $i$, $a_j$ is the detector gain used during scan $j$ and $b_j$ is the scanner offset of scan $j$.

Another embodiment of the invention relates to the computer program above wherein said algorithm of the said computing routine(s), stored on a computer readable medium, comprises a first matrix to determine values of $a_j$ and $b_j$, and a second matrix to determine values of $v_i$, said matrices identical in dimensions to $M_{ij}$ and the said algorithm comprising the following steps:

a) use of equation [1] and the last calculated values of $a_j$, $b_j$ and $v_i$ to predict measured intensity values, and;

b) where $M_{ij}$ is similar to predicted intensity value of step a), set the corresponding element of the first matrix to one, otherwise to zero, said similarity judged using stringent criteria, and;

c) where $M_{ij}$ is similar to predicted intensity value of step a), set the corresponding element of the second matrix to one, otherwise to zero, said similarity judged using less-stringent criteria, and;

d) minimisation of said equation to determine $a_j$ and $b_j$, said minimisation using as input variables $M_{ij}$ and the last-calculated $v_i$, said minimisation excluding or including the measured intensity values determined according to the criteria of b) by comprising a multiplication step involving the corresponding element of the first matrix, and;

e) minimisation of said equation to determine $v_i$, said minimisation using as input variables $M_{ij}$ and the last-calculated values of $a_j$ and $b_j$, said minimisation excluding or including the measured intensity values determined according to the criteria of c) by comprising a multiplication step involving the corresponding element of the second matrix, and;

f) repetition of steps a) to f) until change in $a_j$, $b_j$ and $v_i$ between iterations falls within predefined limits.

Another embodiment of the invention relates to the computer program above wherein step of judging similarity between said predicted intensity values and measured intensity values comprises the following steps:

a) values of $v_i$ are calculated using only columns of $M_{ik}$ for values of k between $k = 1$ and $k = j - 1$, where $j$ ($j>1$) is a column of data, said calculation performed using the iterative-least-squares fitting, result of said calculation is $v_i(j)$, and

b) predicted intensity values are calculated using equation [2] -

$$P_{ij} = a_j v_i(j) + b_j \qquad [2]$$

- in which $a_j$, $v_i(j)$ and $b_j$ are defined above, and $P_{ij}$ is the predicted intensity value of sample $i$, recorded in scan $j$, and

c) using MATRIX $NS_{ij}$, the median error is calculated using equation [3] -

$$ME = \operatorname*{median}_{i \mid NS(i,j)=1} \left( \left| P_{ij} - M_{ij} \right| \right) \qquad [3]$$

- in which $M_{ij}$ and $NS$ and $P_{ij}$ are defined above and $ME$ is the median error, and

d) If $ME$ is greater than 500, $ME$ is set to 500.

e) Using equation [4] -

$$NS_{ij} = P_{ij} < (M_{ij} + d.ME(1+e.NS_{ij}))$$ [4]

- in which $NSij$, $Pij$ and $ME$ are defined above, $d$ is a value in the range 1 to 30, $e$ is a value in the range 0 to 1, element $NSij$ of MATRIX $NSij$ is set to zero if the predicted value is much higher than the real value compared to $ME$, or is set to one otherwise, and

f) Using equation [5] -

$$NS2_{ij} = |P_{ij} - M_{ij}| < f.ME(1 + g.NS2_{ij})$$ [5]

- in which $NSij$, $Pij$ and $ME$ are defined above, $f$ is a value in the range 1 to 30, $g$ is a value in the range 0 to 1, element $NS2ij$ of MATRIX $NS2ij$ is set to zero if the predicted value largely differs from the real value compared to ME, or is set to one otherwise.

Another embodiment of the invention relates to a device comprising an optical detector and a computing routine(s), stored on a computer readable medium, measures the intensities of a set of samples, the said set of samples having a wider dynamic range than that of the detector, the said device performing operations from a list comprising:

a) measurements by the optical detector two or more different detector gains wherein said gains are preferably close to the higher and lower limits for the set of samples, and
b) of the said computing routine, an algorithm comprising iterative least-squares minimisation predicts measured intensity values using an equation relating measured intensity values to the detector gain, the real intensity and constants, and
c) of the said computing routine an algorithm comprising least squares minimisation procedures, removes from the data set all types of saturated data while retaining close-to-saturated data by using an equation
d) of the said computing routine, an algorithm comprising iterative least-squares minimisation procedures determines the real intensity using said equation relating the latter to the detector gain, the measured intensity and constants, and
e) the said real intensity values being presented to the operator as data.

Another embodiment of the invention relates to the device above wherein:

i) the algorithm comprises an iterative routine comprising an equation, said equation relating measured intensity to real intensity and to detector gain using constants, said algorithm comprising the following steps:

a) calculating predicted intensities of samples using said equation and last calculated real intensity values, detector gains and constants, and;
b) using a least-squares minimisation of said equation to determine the detector gain and constants, said minimisation using as input variables the measured intensity values and last-calculated real intensity values, said minimisation also excluding samples judged to have measured intensity values different from the predicted intensity values of step b), said judgment based on stringent criteria.
c) using a least-squares minimisation of said equation to determine the real intensity values, said minimisation using as input variables the measured intensity values and last-calculated detector gain and constants of step b), said minimisation also excluding samples judged to have measured intensity values different from the predicted intensity values of step b), said judgment based on less-stringent criteria.
d) steps a) to d) repeated until change in the constants and real intensity value between iterations is within a predetermined limit.

ii) the said real intensity values being presented to the operator as data.

Another embodiment of the invention relates to the device above wherein the said algorithm comprises equation [1] -

$$M_{ij} = v_i a_j + b_j \qquad [1]$$

- in which $M_{ij}$ is the measured intensity of the sample $i$, measured in scan $j$, $v_i$ is the real intensity of spot $i$, $a_j$ is the detector gain used during scan $j$ and $b_j$ is the scanner offset of scan $j$.

Another embodiment of the invention relates to the device above wherein the said algorithm comprises a first matrix to determine values of $a_j$ and $b_j$, and a second matrix to determine values of $v_i$, said matrices identical in dimensions to $M_{ij}$ and the said algorithm comprising the following steps:

a) use of equation [1] and the last calculated values of $a_j$, $b_j$ and $v_i$ to predict measured intensity values, and;
b) where $M_{ij}$ is similar to predicted intensity value of step a), set the corresponding element of the first matrix to one, otherwise to zero, said similarity judged using stringent criteria, and;
c) where $M_{ij}$ is similar to predicted intensity value of step a), set the corresponding element of the second matrix to one, otherwise to zero, said similarity judged using less-stringent criteria, and;
d) minimisation of said equation to determine $a_j$ and $b_j$, said minimisation using as input variables $M_{ij}$ and the last-calculated $v_i$, said minimisation excluding or including the measured intensity values determined according to the criteria of b) by comprising a multiplication step involving the corresponding element of the first matrix, and;
e) minimisation of said equation to determine $v_i$, said minimisation using as input variables $M_{ij}$ and the last-calculated values of $a_j$ and $b_j$, said minimisation excluding or including the measured intensity values determined according to the criteria of c) by comprising a multiplication step involving the corresponding element of the second matrix, and;
f) repetition of steps a) to f) until change in $a_j$, $b_j$ and $v_i$ between iterations falls within predefined limits.

Another embodiment of the invention relates to the device above wherein the step of judging similarity between said predicted intensity values and measured intensity values comprises the following steps:

a) values of $v_i$ are calculated using only columns of $M_{ik}$ for values of k between $k = 1$ and $k = j - 1$, where $j$ ($j>1$) is a column of data, said calculation performed using the iterative-least-squares fitting, result of said calculation is $v_i(j)$, and

b) predicted intensity values are calculated using equation [2] -

$$P_{ij} = a_j v_i(j) + b_j \qquad [2]$$

- in which $a_j$, $v_i(j)$ and $b_j$ are defined above, and $P_{ij}$ is the predicted intensity value of sample $i$, recorded in scan $j$, and

c) using MATRIX $NS_{ij}$, the median error is calculated using equation [3] -

$$ME = \underset{i \mid NS(i,j)=1}{\mathrm{median}}\left(\left|P_{ij} - M_{ij}\right|\right) \qquad [3]$$

- in which $M_{ij}$ and $NS_{ij}$ and $P_{ij}$ are defined above and $ME$ is the median error, and

d) if $ME$ is greater than 500, $ME$ is set to 500, and

e) using equation [4] -

$$NS_{ij} = P_{ij} < (M_{ij} + d.ME(1 + e.NS_{ij})) \qquad [4]$$

- in which $NS_{ij}$, $P_{ij}$ and $ME$ are defined above, $d$ is a value in the range 1 to 30, $e$ is a value in the range 0 to 1, element $NS_{ij}$ of MATRIX $NS_{ij}$ is set to zero if the predicted value is much higher than the real value compared to $ME$, or is set to one otherwise, and

f) using equation [5] -

$$NS2_{ij} = |P_{ij} - M_{ij}| < f.ME(1 + g.NS2_{ij}) \qquad [5]$$

- in which $NS2ij$, $Pij$ and $ME$ are defined above, $f$ is a value in the range 1 to 30, $g$ is a value in the range 0 to 1, element $NS2ij$ of MATRIX $NS2ij$ is set to zero if the predicted value largely differs from the real value compared to $ME$, or is set to one otherwise.

Another embodiment of the invention relates to data comprising real intensity values, the said data resulting from the transformation of measurements taken from a set of samples using an optical detector, the said set of samples having a wider dynamic range than that of the detector, the said measurements taken at two ore more different detector gains wherein said gains are preferably close to the higher and lower limits for the set of samples, the said transformation performed using a computing routine, comprising iterative least-squares minimisation procedures, removing from the data set all types of saturated data while retaining close-to-saturated data.

**Figures**

Figure 1 is a flow chart demonstrating the initialisation and iterative routines of the invention.

Figure 2 is a flow chart comprising the iterative routine of Figure 1 in addition to two boxes **(Box 18** and **Box 19)** which indicate the points at which normalisation may occur.

Figure 3 shows the results of scanning a microarray of samples using three different gains - G1, G2 and G3 where G1 < G2 < G3 - and highlighting saturated samples which have a measured intensity below the upper known limit of the detector.

Figure 4 shows the surprising result of applying the invention to an array of samples.

Figure 5 shows the results of scanning a microarray of samples using three different gains - G1, G2 and G3 where G1 < G2 < G3 - and highlights a sample which at high gain, is contaminating its background, leading to the sample having a measured intensity which is lower than that measured at a lower gain.

Examples

**Example 1**

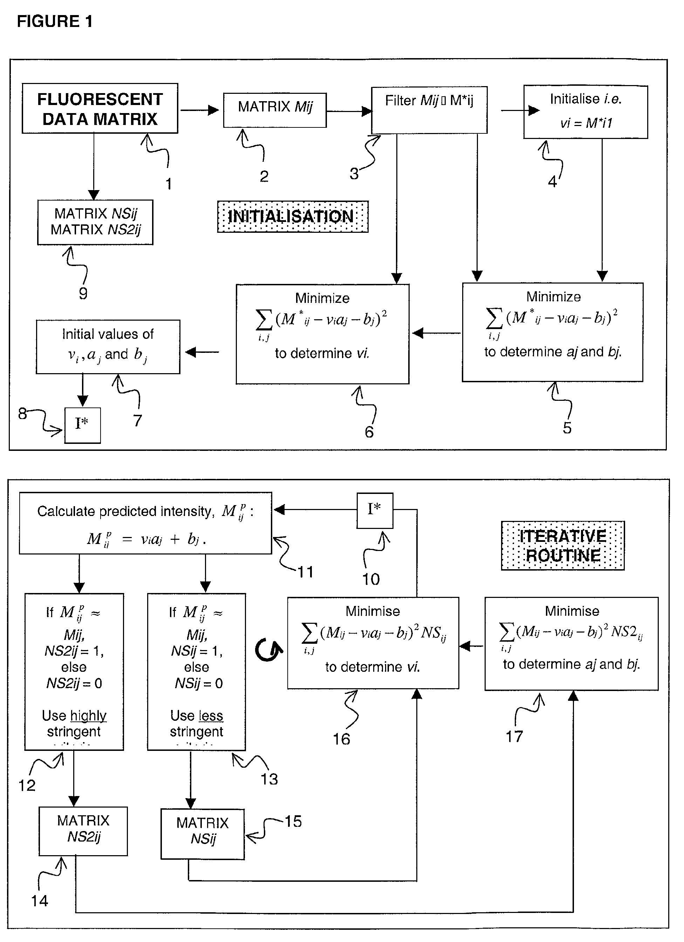

Figure 1 is a flow chart demonstrating the initialisation and iterative routines of the invention. **Box 1** represents measured intensity values recorded for an array of samples at a range of detector gains. In **Box 2,** said the measured intensity are copied to MATRIX $Mij$ where element $Mij$ contains the measured intensity value for spot $i$ *during* scan $j$, as defined above. MATRIX $Mij$ as used in the initialisation procedure is not modified; it is equal and equivalent to MATRIX $Mij$ used in the iterative routine. **Box 3** of the routine is a filtering step which removes all measurements of said Box 2 lying at the known limit of the detector (*e.g.* removes $Mij > 55000$), to produce a matrix of filtered values of $M*ij$ **(Box 3).** The filtering step of Box 3 is optional. In **Box 4,** initial values of the $vi$ (wherein $vi$ are the real intensity values as defined above), required to initialise the main iterative routine, are set to the values $M*ij$ measured with the lowest detector gain *i.e.* $vi = M*i1$. In **Box 5,** using the initialised values of $vi$ described above, and the values of $M*ij$ (Box 3), values of $aj$ and $bj$ (where in $aj$ is the detector gain at scan $j$, and $bj$ is a constant) are determined by the least squares minimisation of the equation given in Box 5. In **Box 6,** using the values of $aj$ and $bj$ determined from Box 5, and the values of $M*ij$ from Box 3, the values of $vi$ are determined by the least squared minimisation of the equation given in Box 6. The so-calculated initial values of $aj$, $bj$ and $vi$ **(Box 7)** are used only in the first iteration of the main routine, and are introduced into said routine as I* **(Box 8).** In **Box 9,** 2 copies of the fluorescent data matrix are made: MATRIX $NSij$ and MATRIX $NS2ij$. Thus, boxes 1 to 9 comprise the initialisation of the iterative routine.

In **Box 10,** the initializing values of $aj$, $bj$ and vi, (I*) are used to predict the measured intensity of each sample using the equation of **Box 11.** The predicted values ($M_{ij}^{p}$) are compared against the experimentally determined measured intensity values held in MATRIX $Mij$ in two comparison procedures of Boxes 12 and 13. In **Box 13,** if the experimentally determined value for the measured intensity, $Mij$, differs significantly from that calculated in Box 11 ($M_{ij}^{p}$), the element $NSij$ held in MATRIX $NSij$ **(Box 15)** is set to zero; otherwise the said element $NSij$ is set to 1. In **Box 12,** if the experimentally determined value for the measured intensity, $Mij$, differs significantly from that calculated in Box 11 ($M_{ij}^{p}$), the element $NS2ij$ held in MATRIX $NS2ij$ **(Box 14)** is set to zero; otherwise the said element $NS2ij$ is set to 1. The criteria for selection is more stringent in **Box 12** than it is in **Box 13,** *i.e.* more elements of $NS2ij$ are set to zero in MATRIX $NS2ij$ than are in MATRIX $NSij$. The former is required to be more stringent because MATRIX $NS2ij$ is used to determine constants in the equation of Box 17 which should be accurately determined. Box 13 is required to be less stringent because MATRIX *NSij* is used to determine the values of *vi,* of which there should be a maximal number. The values held in modified MATRIX *NS2ij* (Box 14), the values held in unmodified MATRIX *Mij* and the value of *vi* determined in Box 16 (or Box 10 for the first iteration) are used as constants for the minimisation of the equation in **Box 17.** The result of box 17 is new values of *aj* and *bj*. The values held in modified MATRIX *NSij* (Box 15), the values held in unmodified MATRIX *Mij,* and the values of *ai* and *bj* determined in Box 17 are used as constants for the minimisation of the equation in **Box 16.** The values of *aj*, *bj* and *vi* calculated in boxes 16 and 17 are used to predict the measured intensity of each sample (Box 11). Box 10 is not applied after the first iteration.

The iterative routine in Figure 1 continues until the change in *aj, bj,* and *vi* between successive iterations is within predetermined limits.

**Example 2**

Boxes 9 and 10 in Figure 1 show that stringent or relaxed criteria respectively are used to determine which measured intensity values are considered for calculations. In determining $a_j$ and $b_j$, stringent criteria are used to remove those measured intensity values from matrix NS2 which fall outside the predicted limits (outliers). Said values are not necessarily all saturated; they are values which show a larger error than expected. The result is a robust linear fit derived from accurate values of *Mij.*

In determining $v_i$, only saturated measured intensity values are removed from matrix NS; those values which fall outside the predicted limits AND whose measurements at higher gains are lower than would be expected are removed. The result is that all non-saturated data is retained, even if said data presents some error.

Saturated measured intensity values are those for which the value at the higher gain is significantly lower than would be expected by using equation [1].

In equation [2] -

$$M_{ij} < a_j v_i + b_j \text{ -delta} \qquad\qquad [2]$$

- wherein *Mij, aj*, *vi* and *bj* are defined above and *delta* is the difference which is the minimum taken to be significant, the goal is to determine *delta* so that normal variations are kept while outliers are correctly detected. The values in the scan with the lowest gain (*j* = 1) are considered as always non-saturated.

One example of the implementation of the selection criteria comprises the following steps:
for each *j* > 1 (for each column of M except the first)

i) Calculate the value of $v_i$ that would be obtained using only the columns $M_{ik}$ for the values of k between *k* = 1 and *k = j* - 1 where *j* (*j* > 1) is a column of data. This is performed by minimising the equation $M_{ik} = v_i a_k + b_k$ as described above. Let the result be $v_i(j)$.

ii) Calculate the predicted value $P_{ij}$ according to equation [3] -

$$P_{ij} = a_j v_i(j) + b_j \qquad\qquad [3]$$

- wherein *aj, vi(j)* and *bj* are defined above and *Pij* is the predicted measured intensity value.

iii) Using the measured intensity values which are non-saturated (in the sense of NS), the median error is calculated in equation [4] -

$$ME = \operatorname*{median}_{i|NS(i,j)=1}\left(\left|P_{ij} - M_{ij}\right|\right) \qquad\qquad [4]$$

wherein *Pij* and *Mij* are defined above, and *ME* is the median error.

iv) If *ME* is too high (higher than 500, for instance), let *ME* = 500. This is done in order to avoid local minima in the beginning.

v) The element *NSij* of MATRIX *NSij* is set to zero if the predicted value is much higher than the real value compared to ME, and is set to one otherwise. Thus, MATRIX *NSij* is modified according to equation [5] -

$$NS_{ij} = P_{ij} < (10.ME(1 + 0.1.NS_{ij})) \tag{6}$$

wherein *NSij, Pij,* and *ME* are defined above.

The term $(1 + 0.1*NS_{ij})$ is used to introduce an hysteresis, so that the measured intensity values close to the limit between saturation and non-saturation stay on one side of the border.

vi) The element *NS2ij* of MATRIX *NS2ij* is set to zero if the predicted value is largely different from the real value, compared to *ME,* and set to one otherwise. Thus, MATRIX *NS2ij* is modified according to equation [6] -

$$NS2_{ij} = |P_{ij} - M_{ij}| < (M_{ij} + 6.ME(1+0.1.NS2_{ij})) \tag{6}$$

wherein *NS2ij, Pij,* and *ME* are defined above.

The criterion is more stringent for MATRIX *NS2ij* than for MATRIX *NSij* because outliers are a burden for the determination of a and b anyway.

The main difference between the two criteria is that the first is symmetrical (all outliers are removed from MATRIX *NS2ij*), while the second is not (only outliers on one side are removed from MATRIX *NSij*). The difference in stringency is in some respects less important; the algorithm can work with both tests using the same stringency. The difference in symmetry, on the other hand, provides a robust screen for outliers in MATRIX *NS2ij.*

Note:

1. The choices of the number 6 and 10 are arbitrary, the algorithm would work with other choices. With lower numbers, it would consider more spot as saturated, with a higher number the opposite, but its behavior would remain similar.

2. Other, simpler, means to detect saturated spots can also be used. For instance, all spots with a measured intensity over a certain threshold (for instance 40,000) could be considered as saturated once and for all. The issue with this simplification is that the threshold must be sufficiently low to encompass all saturated spots, which means that many well-measured spots are considered as saturated. Also it is always possible that a spot saturate at a lower intensity.

**Example 3**

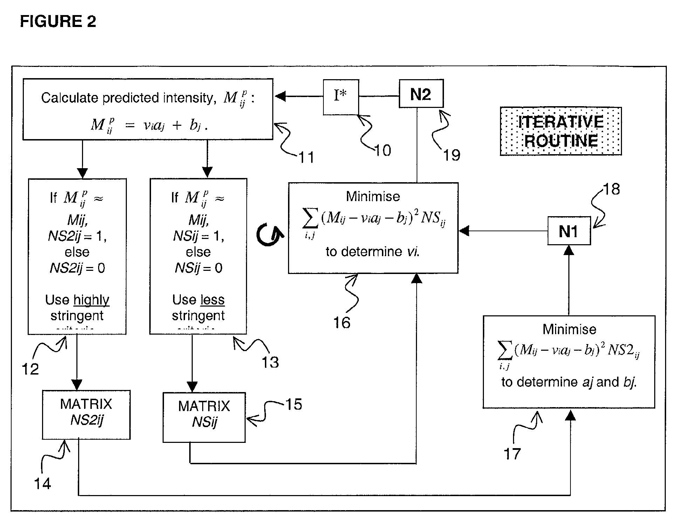

Figure 2 introduces a normalisation procedures (N1, **Box 18** and N2, **Box 19**) into the iterative routine in order to reduce the number of iterations required to reach the solution. Any normalisation procedure which satisfies the criteria described below is part of the invention.

The algorithm of the iterative routine minimises the error of the equation [7] -

$$\sum_{i,j} \left(M_{ij} - v_i a_j - b_j\right)^2 NS_{ij} \tag{7}$$

wherein *Mij, vi, aj, bj* and *NSij* are defined above.

If, for example, the values of *a, b* and *v* have been found which minimize [7], and *x* = *ka* and *y* = *v*/*k,* where *k* is any number other than zero and one. Then equation [8] is true -

$$\sum_{i,j} \left(M_{ij} - y_i x_j - b_j\right)^2 NS_{ij} = \sum_{i,j} \left(M_{ij} - \frac{v_i}{k} ka_j - b_j\right)^2 NS_{ij} = \sum_{i,j} \left(M_{ij} - v_i a_j - b_j\right)^2 NS_{ij} \tag{8}$$

wherein *Mij, vi, aj, bj, NSij, x, y* and *k* are defined above.

Hence *x* and *y* minimize [7] also. This means that the solution should be constrained in order to remove this degree of freedom. This can be done by setting $a_1$=1, for instance by minimizing [7] only on $a_2$-$a_n$, considering that $a_1$=1. It is however more efficient to solve [7] for $a_1$-$a_n$, and then correct $a$ by dividing it by $a_1$.

In the case that $\textbf{x} = \textbf{b} + k\textbf{a}$ and $\textbf{y} = \textbf{v} - k$, where $k$ is any number different than zero. Then equation [9] is true -

$$\sum_{i,j}\left(M_{ij} - y_i a_j - x_j\right)^2 NS_{ij} = \sum_{i,j}\left(M_{ij} - \left(v_i - k\right).a_j - b_j - ka_j\right)^2 NS_{ij} = \sum_{i,j}\left(M_{ij} - v_i a_j - b_j\right)^2 NS_{ij}$$

[9]

wherein *Mij, vi, aj, bj, NSij, x, y* and *k* are defined above.

Hence x and y minimize [7] also. This means that the solution should be constrained in order to remove this degree of freedom, for instance by setting min($v$)=0. This can be done efficiently by correcting $v$ and $b$ using the formulas given. Other choices could be made as well: for instance, it would be possible to decide that $b_1$=0, and use a similar technique to normalize.

Because the normalisations are arbitrary, it is possible to change the results after convergence from one choice of normalisation to any other choice, for instance from the min($v$)=0 to the $b_1$=0 normalization.

In one example of a normalisation procedure, the following parameters are normalised once per cycle of the iterative routine of Figure 2. Figure 2 comprises the iterative routine of Figure 1 in addition to two boxes (**Box 18** and **Box 19**) which indicate the points at which normalisation may occur. The possible stages at which normalisation occurs is not limited to those indicated in Figure 2. The stages referred to in Figure 2 are the preferred positions for normalisation using the equations below. Other normalization methods which satisfy the critereia described above may be inserted at positions in addition to those indicated in Figure 2. Examples of normalisation equations are given:

$$a_j = \frac{a_j}{a_1} \tag{10}$$

$$b_j = b_j - a_j b_1 \tag{11}$$

$$b_j = b_j + \min(v).a_j \tag{12}$$

$$v_i = v_i - \min(v) \tag{13}$$

$$a_j = \frac{a_j}{a_2} \tag{14}$$

$$b_j = b_j - a_j b_2 \tag{15}$$

In one example of a normalisation procedure, the parameters are normalised once per cycle of the iterative routine of Figure 2. N1, **(Box 18)** normalises both the values of *aj* and *bj* according to equations [10] and [11] respectively above, and N2 **(Box 19)** performs no operation.

In another example of a normalisation procedure, the parameters are normalised once per cycle of the iterative routine of Figure 2. N1, **(Box 18)** normalises both the values of *aj* and *bj* according to equations **[14]** and **[15]** respectively above, and **N2 (Box 19)** performs no operation.

In yet another example of a normalisation procedure, the parameters are normalised once per cycle of the iterative routine of Figure 2. N1 **(Box 18)** normalises the value of *aj* according to equation [10] above, and N2 **(Box 19)** normalises the values of both *vi* and *bj* according to equations [12] and [13] respectively above.

**Example 4**

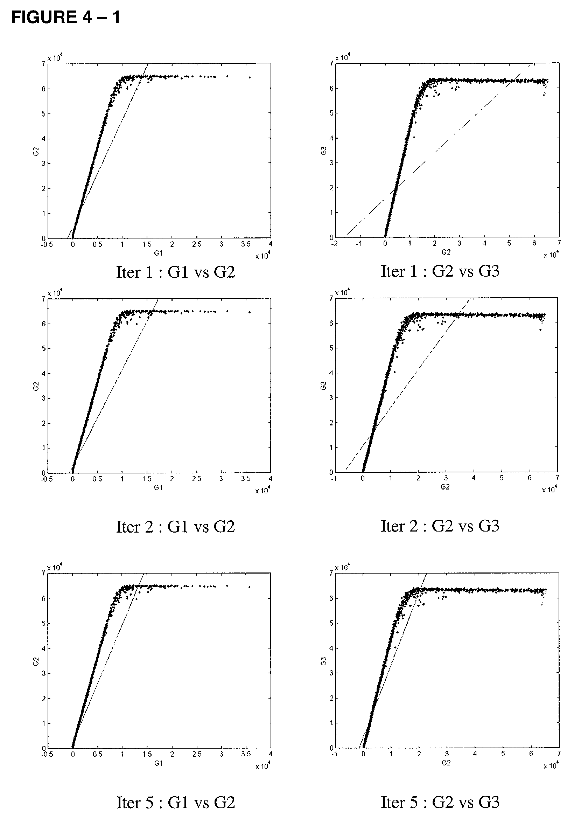

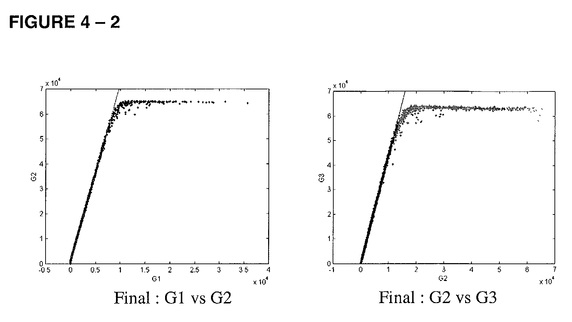

Figure 4 shows the surprising result of applying the invention to an array of samples. The samples were measured at 3 different detector gains (G1 < G2 < G3) and the straight line represents equation [1]. The figure clearly demonstrates the algorithm moving towards values of $vi$, $aj$ and $bj$ which match the real intensity values as saturated data is discarded. The final fit matches only non-saturated data.

**Claims**

1. A method to quantitate a set of samples using an optical reader, the dynamic range of said set of samples being greater than that of the said optical reader, by measuring intensities of said set at two or more detector gains wherein said gains are preferably close to the higher and lower limits for the said set, and by analysing said measured intensity values using an algorithm to discard all types of saturated sample data, retain sample data close to detector-saturation and calculate real intensity values for each sample.

2. A method according to claim 1 wherein the said algorithm comprises iterative least-squares minimisations to predict said measured intensity values.

3. A method according to any of claims 1 or 2 wherein said algorithm comprises a comparison procedure to discard all types of saturated data and/or non-predicted data from calculations to determine the real intensity values for each sample.

4. A method according to claims 1 to 3 wherein the algorithm comprises an iterative routine comprising an equation, said equation relating measured intensity to real intensity and to detector gain using constants, said algorithm comprising the following steps:

    a) calculating predicted intensities of samples using said equation and last calculated real intensity values, detector gains and constants, and;
    b) using a least-squares minimisation of said equation to determine the detector gain and constants, said minimisation using as input variables the measured intensity values and last-calculated real intensity values, said minimisation also excluding samples judged to have measured intensity values different from the predicted intensity values of step b), said judgment based on stringent criteria.
    c) using a least-squares minimisation of said equation to determine the real intensity values, said minimisation using as input variables the measured intensity values and last-calculated detector gain and constants of step b), said minimisation also excluding samples judged to have measured intensity values different from the predicted intensity values of step b), said judgment based on less-stringent criteria.
    d) steps a) to d) repeated until change in the constants and real intensity value between iterations is within a predetermined limit.

5. A method according to any of claims 1 to 4 wherein the said algorithm comprises equation [1] -

$$M_{ij} = v_i a_j + b_j \qquad [1]$$

    - in which $Mij$ is the measured intensity of the sample $i$, measured in scan $j$, $vi$ is the real intensity of sample $i$, $aj$ is the detector gain used during scan $j$ and $bj$ is the scanner offset of scan $j$.

6. A method according to any of claims 1 to 5 wherein the said algorithm comprises a first matrix to determine values of $aj$ and $bj$, and a second matrix to determine values of $vi$, said matrices identical in dimensions to $Mij$ and the said algorithm comprising the following steps:

    a) use of equation [1] and the last calculated values of $aj$, $bj$ and $vi$ to predict measured intensity values, and;
    b) where $Mij$ is similar to predicted intensity value of step a), set the corresponding element of the first matrix to one, otherwise to zero, said similarity judged using stringent criteria, and;
    c) where $Mij$ is similar to predicted intensity value of step a), set the corresponding element of the second matrix to one, otherwise to zero, said similarity judged using less-stringent criteria, and;
    d) least-squares minimisation of said equation to determine $aj$ and $bj$, said minimisation using as input variables $Mij$ and the last-calculated $vi$, said minimisation excluding or including the measured intensity values determined according to the criteria of b) by comprising a multiplication step involving the corresponding element of the first matrix, and;

e) least-squares minimisation of said equation to determine *vi,* said minimisation using as input variables *Mij* and the last-calculated values of *aj* and *bj,* said minimisation excluding or including the measured intensity values determined according to the criteria of c) by comprising a multiplication step involving the corresponding element of the second matrix, and;

f) repetition of steps a) to f) until change in *aj, bj* and *vi* between iterations falls within predefined limits.

7. A method according to claim 6 wherein initial values of *aj, bj* and *vi* are calculated using an initialisation routine comprising the following steps:

a) measured intensity values, *Mij,* are optionally filtered to remove those at the known limit for the detector and are used in the proceeding steps, and

b) initial values of the real intensity *vi,* are set to the of values *Mij* measured with the lowest detector gain *i.e.* $vi = M_{i1}$, and

c) initial values of *vi* from step b), and the values of *Mij* are used to calculate the values of *aj* and *bj* by the least-squares minimisation of the equation [1], and

d) the values of *aj* and *bj* calculated in step c) and the values of *Mij* are used to calculate the values of *vi* by the least-squares minimisation of the equation [1], and

e) the so-calculated initial values of *aj, bj* and *vi* are used in the first iteration of the iterative routine.

8. A method according to any of claims 5 to 7 wherein the values of *aj* and *bj* determined during the said iterative routine are normalised after the first iteration and each subsequent iteration by dividing *aj* by $a_1$ and by substracting $aj.b_1$ from *bj,* respectively.

9. A method according to any of claims 5 to 7 wherein the values of *aj, bj* and *vi* determined during the said iterative-routine are normalised after the first and each subsequent iteration by dividing *aj* by $a_1$, and by adding *bj* to min (*v*).*aj*, and by substracting min(*v*) from *vi,* respectively.

10. A method according to any of claims 6 to 9 wherein step of judging similarity between said predicted intensity values and measured intensity values comprises the following steps:

a) values of *vi* are calculated using only columns of *Mik* for values of k between $k = 1$ and $k = j - 1$, where *j* (*j*>1 ) is a column of data, said calculation performed using the iterative-least-squares fitting, result of said calculation is *vi(j),* and

b) Predicted intensity values are calculated using equation [2] -

$$P_{ij} = a_j\, v_i(j) + b_j \qquad\qquad [2]$$

- in which *aj, vi(j)* and *bj* are defined above, and *Pij* is the predicted intensity value of sample *i*, recorded in scan *j*, and

c) Using MATRIX *NSij,* the median error is calculated using equation [3]-

$$ME \;=\; \operatorname*{median}_{i\,\mid NS(i,\,j)=1}\!\left(\left|P_{ij} - M_{ij}\right|\right) \qquad\qquad [3]$$

- in which *Mij* and *NSij* and *Pij* are defined above and *ME* is the median error, and

d) If *ME* is greater than 500, *ME* is set to 500, and

e) Using equation [4] -

$$NS_{ij} = P_{ij} < (M_{ij} + d.ME(1 + e.NS_{ij})) \qquad\qquad [4]$$

- in which *NSij, Pij* and *ME* are defined above, *d* is a value in the range 1 to 30, *e* is a value in the range 0 to 1, element *NSij* of MATRIX *NSij* is set to zero if the predicted value is much higher than the real value compared to *ME,* or is set to one otherwise, and

f) Using equation [5] -

$$NS2_{ij} = |P_{ij} - M_{ij}| < f.ME(1 + g.NS2_{ij}) \qquad [5]$$

- in which *NS2ij, Pij* and *ME* are defined above, *f* is a value in the range 1 to 30, *g* is a value in the range 0 to 1, element *NS2ij* of MATRIX *NS2ij* is set to zero if the predicted value largely differs from the real value compared to ME, or is set to one otherwise.

**11.** A method according to any of claims 1 to 10 wherein said optical reader is a fluorescence micro-array scanner.

**12.** A method according to any of claims 1 to 11 wherein the said samples are fluorescently labelled DNA probes hybridized onto cDNA attached to a solid support.

**13.** A method according to any of claims 1 to 12 comprising the use of a computer program comprising a computing routine(s), stored on a computer readable medium for transforming a set of wide dynamic-range sample data measured by optical means at two or more different detector gains, said gains preferably close to the higher and lower limits for the set of samples, the said data held as a file in any format and/or as data passed from another computing routine(s), using the said algorithm, to a new data set that corresponds to real intensity values, the results given as readable output and/or a file of any format and/or data passed to other computing routine(s).

**14.** A computer program comprising a computing routine(s), stored on a computer readable medium for transforming a set of wide dynamic-range sample data measured by optical means at two or more different detector gains, said gains preferably close to the higher and lower limits for the set of samples, the said data held as a file in any format and/or as data passed from another computing routine(s), using an algorithm, the said algorithm removing all types of saturated sample data, retaining close-to-saturated sample data and calculating real intensity values for each sample, the said transformation resulting in a new data set that corresponds to real intensity values, the results given as readable output and/or a file of any format and/or data passed to other computing routine(s).

**15.** A computer program according to claim 14 wherein said algorithm of the said computing routine(s), stored on a computer readable medium, comprises an iterative routine comprising an equation, said equation relating measured intensity to real intensity and to detector gain using constants, said algorithm comprising the following steps:

a) calculating predicted intensities of samples using said equation and last calculated real intensity values, detector gains and constants, and;

b) using a least-squares minimisation of said equation to determine the detector gain and constants, said minimisation using as input variables the measured intensity values and last-calculated real intensity values, said minimisation also excluding samples judged to have measured intensity values different from the predicted intensity values of step b), said judgment based on stringent criteria.

c) using a least-squares minimisation of said equation to determine the real intensity values, said minimisation using as input variables the measured intensity values and last-calculated detector gain and constants of step b), said minimisation also excluding samples judged to have measured intensity values different from the predicted intensity values of step b), said judgment based on less-stringent criteria.

d) steps a) to d) repeated until change in the constants and real intensity value between iterations is within a predetermined limit.

**16.** A computer program according to any of claims 14 and 15 wherein the said equation of said algorithm of the said computing routine(s), stored on a computer readable medium takes the form of equation [1] -

$$M_{ij} = v_i a_j + b_j \qquad [1]$$

in which *Mij* is the measured intensity of the sample *i,* measured in scan *j* (for scans *j* = 1 to n, where the scan j

=1 has the lowest detector gain and $j$ = n has the highest detector gain), $vi$ is the real intensity of spot $i$, $aj$ is the detector gain used during scan $j$ and $bj$ is the scanner offset of scan $j$.

**17.** A computer program according to any of claims 14 to 16 wherein said algorithm of the said computing routine(s), stored on a computer readable medium, comprises a first matrix to determine values of $aj$ and $bj$, and a second matrix to determine values of $vi$, said matrices identical in dimensions to $Mij$ and the said algorithm comprising the following steps:

a) use of equation [1] and the last calculated values of $aj$, $bj$ and $vi$ to predict measured intensity values, and;
b) where $Mij$ is similar to predicted intensity value of step a), set the corresponding element of the first matrix to one, otherwise to zero, said similarity judged using stringent criteria, and;
c) where $Mij$ is similar to predicted intensity value of step a), set the corresponding element of the second matrix to one, otherwise to zero, said similarity judged using less-stringent criteria, and;
d) minimisation of said equation to determine $aj$ and $bj$, said minimisation using as input variables $Mij$ and the last-calculated $vi$, said minimisation excluding or including the measured intensity values determined according to the criteria of b) by comprising a multiplication step involving the corresponding element of the first matrix, and;
e) minimisation of said equation to determine $vi$, said minimisation using as input variables $Mij$ and the last-calculated values of $aj$ and $bj$, said minimisation excluding or including the measured intensity values determined according to the criteria of c) by comprising a multiplication step involving the corresponding element of the second matrix, and;
f) repetition of steps a) to f) until change in $aj$, $bj$ and $vi$ between iterations falls within predefined limits.

**18.** A computer program according to any of claims 14 to 17 wherein step of judging similarity between said predicted intensity values and measured intensity values comprises the following steps:

a) values of $vi$ are calculated using only columns of $Mik$ for values of k between $k = 1$ and $k = j - 1$, where $j$ ($j>1$) is a column of data, said calculation performed using the iterative-least-squares fitting, result of said calculation is $vi(j)$, and

b) predicted intensity values are calculated using equation [2] -

$$P_{ij} = a_j v_i(j) + b_j \qquad\qquad [2]$$

- in which $aj$, $vi(j)$ and $bj$ are defined above, and $Pij$ is the predicted intensity value of sample $i$, recorded in scan $j$, and

c) using MATRIX $NSij$, the median error is calculated using equation [3] -

$$ME = \underset{i\,|\,NS(i,j)=1}{\mathrm{median}}\left(\left|P_{ij} - M_{ij}\right|\right) \qquad\qquad [3]$$

- in which $Mij$ and $NS$ and $Pij$ are defined above and $ME$ is the median error, and

d) If $ME$ is greater than 500, $ME$ is set to 500.

e) Using equation [4] -

$$NS_{ij} = P_{ij} < (M_{ij} + d.ME(1 + e.NS_{ij})) \qquad\qquad [4]$$

- in which $NSij$, $Pij$ and $ME$ are defined above, $d$ is a value in the range 1 to 30, $e$ is a value in the range 0 to 1, element $NSij$ of MATRIX $NSij$ is set to zero if the predicted value is much higher than the real value compared to $ME$, or is set to one otherwise, and

f) Using equation [5] -

$$NS2_{ij} = |P_{i_j} - M_{ij}| < f.ME(1+g.NS2_{ij}) \tag{5}$$

- in which *NS2ij, Pij* and *ME* are defined above, *f* is a value in the range 1 to 30, *g* is a value in the range 0 to 1, element *NS2ij* of MATRIX *NS2ij* is set to zero if the predicted value is largely different from the real value compared to ME, or is set to one otherwise.

**19.** A device comprising an optical detector and a computing routine(s), stored on a computer readable medium, measures the intensities of a set of samples, the said set of samples having a wider dynamic range than that of the detector, the said device performing operations from a list comprising:

a) measurements by the optical detector two or more different detector gains wherein said gains are preferably close to the higher and lower limits for the set of samples, and
b) of the said computing routine, an algorithm comprising iterative least-squares minimisation predicts measured intensity values using an equation relating measured intensity values to the detector gain, the real intensity and constants, and
c) of the said computing routine an algorithm comprising least squares minimisation procedures, removes from the data set all types of saturated data while retaining close-to-saturated data by using an equation
d) of the said computing routine, an algorithm comprising iterative least-squares minimisation procedures determines the real intensity using said equation relating the latter to the detector gain, the measured intensity and constants, and
e) the said real intensity values being presented to the operator as data.

**20.** A device according to claim 19 wherein:

i) the algorithm comprises an iterative routine comprising an equation, said equation relating measured intensity to real intensity and to detector gain using constants, said algorithm comprising the following steps:

a) calculating predicted intensities of samples using said equation and last calculated real intensity values, detector gains and constants, and;
b) using a least-squares minimisation of said equation to determine the detector gain and constants, said minimisation using as input variables the measured intensity values and last-calculated real intensity values, said minimisation also excluding samples judged to have measured intensity values different from the predicted intensity values of step b), said judgment based on stringent criteria.
c) using a least-squares minimisation of said equation to determine the real intensity values, said minimisation using as input variables the measured intensity values and last-calculated detector gain and constants of step b), said minimisation also excluding samples judged to have measured intensity values different from the predicted intensity values of step b), said judgment based on less-stringent criteria.
d) steps a) to d) repeated until change in the constants and real intensity value between iterations is within a predetermined limit.

ii) the said real intensity values being presented to the operator as data.

**21.** A device according to claims 19 or 20 wherein the said algorithm comprises equation [1 ] -

$$M_{ij} = v_i a_j + b_j \tag{1}$$

- in which *Mij* is the measured intensity of the sample *i,* measured in scan *j, vi* is the real intensity of sample *i, aj* is the detector gain used during scan *j* and *bj* is the scanner offset of scan *j.*

**22.** A device according to claims 19 to 21 wherein the said algorithm comprises a first matrix to determine values of *aj* and *bj,* and a second matrix to determine values of *vi,* said matrices identical in dimensions to *Mij* and the said algorithm comprising the following steps:

a) use of equation [1] and the last calculated values of *aj, bj* and *vi* to predict measured intensity values, and;

b) where *Mij* is similar to predicted intensity value of step a), set the corresponding element of the first matrix to one, otherwise to zero, said similarity judged using stringent criteria, and;

c) where *Mij* is similar to predicted intensity value of step a), set the corresponding element of the second matrix to one, otherwise to zero, said similarity judged using less-stringent criteria, and;

d) minimisation of said equation to determine *aj* and *bj,* said minimisation using as input variables *Mij* and the last-calculated *vi,* said minimisation excluding or including the measured intensity values determined according to the criteria of b) by comprising a multiplication step involving the corresponding element of the first matrix, and;

e) minimisation of said equation to determine *vi,* said minimisation using as input variables *Mij* and the last-calculated values of *aj* and *bj,* said minimisation excluding or including the measured intensity values determined according to the criteria of c) by comprising a multiplication step involving the corresponding element of the second matrix, and;

f) repetition of steps a) to f) until change in *aj, bj* and *vi* between iterations falls within predefined limits.

23. A device according to any of claims 19 to 22 wherein step of judging similarity between said predicted intensity values and measured intensity values comprises the following steps:

a) values of *vi* are calculated using only columns of *Mik* for values of k between $k = 1$ and $k = j - 1$, where *j* (j>1) is a column of data, said calculation performed using the iterative-least-squares fitting, result of said calculation is *vi(j),* and

b) predicted intensity values are calculated using equation [2] -

$$P_{ij} = a_j v_i(j) + b_j \qquad\qquad [2]$$

- in which *aj, vi(j)* and *bj* are defined above, and *Pij* is the predicted intensity value of sample *i*, recorded in scan *j*, and

c) using MATRIX *NSij,* the median error is calculated using equation [3] -

$$ME = \underset{i\,|\,NS(i,j)=1}{\mathrm{median}}\left(\left|P_{ij} - M_{ij}\right|\right) \qquad\qquad [3]$$

- in which *Mij* and *NS* and *Pij* are defined above and *ME* is the median error, and

d) if *ME* is greater than 500, *ME* is set to 500, and

e) using equation [4] -

$$NS_{ij} = P_{ij} < (M_{ij} + d.ME(1 + e.NS_{ij})) \qquad\qquad [4]$$

- in which *NSij, Pij* and *ME* are defined above, *d* is a value in the range 1 to 30, *e* is a value in the range 0 to 1, element *NSij* of MATRIX *NSij* is set to zero if the predicted value is much higher than the real value compared to *ME,* or is set to one otherwise, and

f) using equation [5] -

$$NS2_{ij} = |P_{ij} - M_{ij}| < f.ME(1 + g.NS2_{ij}) \qquad\qquad [5]$$

- in which *NS2ij, Pij* and *ME* are defined above, *f* is a value in the range 1 to 30, *g* is a value in the range 0 to 1, element *NS2ij* of MATRIX *NS2ij* is set to zero if the predicted value largely differs from the real value compared to *ME,* or is set to one otherwise.

**24.** Data comprising real intensity values, the said data resulting from the transformation of measurements taken from a set of samples using an optical detector, the said set of samples having a wider dynamic range than that of the detector, the said measurements taken at two or more different detector gains wherein said gains are preferably close to the higher and lower limits for the set of samples, the said transformation performed using a computing routine, comprising iterative least-squares minimisation procedures, removing from the data set all types of saturated data while retaining close-to-saturated data.

**FIGURE 1**

**FIGURE 2**

**FIGURE 3**

# FIGURE 4 – 1

Iter 1 : G1 vs G2

Iter 1 : G2 vs G3

Iter 2 : G1 vs G2

Iter 2 : G2 vs G3

Iter 5 : G1 vs G2

Iter 5 : G2 vs G3

**FIGURE 4 – 2**

Final : G1 vs G2          Final : G2 vs G3

**FIGURE 5**

**European Patent Office**

# EUROPEAN SEARCH REPORT

Application Number

EP 02 44 7151

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | US 6 171 793 B1 (TRAN HUU MINH ET AL) 9 January 2001 (2001-01-09) * abstract * * figure 12 * * column 4, line 25 - column 5, line 10 * * column 9, line 61 - line 67 * * claim 11 * --- | 1-10 | G06F19/00 |
| A | HSIAO L., ET AL.: "Correcting for signal saturation errors in the analysis of microarray data" BIOTECHNIQUES, vol. 32, no. 2, February 2002 (2002-02), pages 330-336, XP001156742 USA ISSN: 0736-6205 * page 330 - page 336 * ----- | 1-10 | |

**TECHNICAL FIELDS SEARCHED (Int.Cl.7)**

G06F

~~The present search report has been drawn up for all claims~~

| Place of search | Date of completion of the search | Examiner |
|---|---|---|
| MUNICH | 13 January 2004 | Samulowitz, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent
Office

Application Number

EP 02 44 7151

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☒ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

**ANNEX TO THE EUROPEAN SEARCH REPORT
ON EUROPEAN PATENT APPLICATION NO.**                EP 02 44 7151

This annex lists the patent family members relating to the patent documents cited in  the above-mentioned European search report.
The members are as contained in the European Patent Office EDP file on
The European Patent Office is in no way liable for these particulars which are merely  given for the purpose of information.

13-01-2004

| Patent document cited in search report | | Publication date | Patent family member(s) | | Publication date |
|---|---|---|---|---|---|
| US 6171793 | B1 | 09-01-2001 | AU | 4343000 A | 02-11-2000 |
| | | | CA | 2370947 A1 | 26-10-2000 |
| | | | EP | 1173616 A1 | 23-01-2002 |
| | | | WO | 0063442 A1 | 26-10-2000 |

EPO FORM P0459